# EUROPEAN PATENT SPECIFICATION

(11) **EP 3 926 315 B1**
(45) Date of publication and mention of the grant of the patent: **07.08.2024**
(21) Application number: 21179753.5
(22) Date of filing: 16.06.2021
(51) Int. Cl.: G01K 11/12, G01K 13/20

(54) **CONTACT THERMOGRAPHY KIT**
KONTAKTTHERMOGRAFIESATZ
KIT DE THERMOGRAPHIE DE CONTACT

(30) Priority: 19.06.2020 IT 202000014707
(43) Date of publication of application: 22.12.2021
(73) Proprietor: I.P.S. International Products & Services S.r.l., 20097 S. Donato Milanese (IT)
(72) Inventor: PASOTTI, Filippo, 20097 San Donato Milanese (MI) (IT)
(74) Representative: Belloni, Giancarlo

(56) References cited:
- US-A- 3 704 625
- US-A- 5 776 074
- IPS PROFESSIONAL AESTHETICS: "Analisi termografica della cellulite", 15 September 2016 (2016-09-15), pages 1 - 4, XP054981503, Retrieved from the Internet <URL:https://www.youtube.com/watch?v=J6h0iGopMDE> [retrieved on 20210309]

## Description

### FIELD OF APPLICATION

The present invention relates to a contact thermography kit.

In particular, the present invention relates to a contact thermography kit for analysing imperfections on the human body.

### PRIOR ART

Nowadays, unsightly effects are known to occur on the human body as a result of incorrect nutrition and of many other factors.

The most common and studied are related to cellulite.

Cellulite is an alteration of the skin tissue which, starting from the fat layer, i.e., the deepest area of the skin, gradually extends to the upper layers, dermis and epidermis.

On the other hand, problems of diffuse or concentrated adiposity have now also become very frequent, even among populations historically only minimally affected by this problem.

Adiposity is an enlargement of fat cells that can affect the whole body in a general way (general adiposity resulting in overweight) or can be localised to certain areas, depending on predisposition and body structure (localised adiposity).

General adiposity and therefore overweight is almost always accompanied by cellulite, but the opposite is not so common; cellulite also affects many slim or even underweight women.

In addition, women and men rely on specialists in order to undertake targeted treatments to improve the imperfections deriving from cellulite and adiposity. However, the effectiveness of these treatments depends on the ability to classify the stages of the imperfection under examination.

On 17 September 2016, the Applicant published a video (https://www.youtube.com/watch?v=J6h0iGopMDE) called *Analisi termografica della cellulite*, i.e. *Thermographic analysis of cellulite*, showing a contact thermography kit similar to the one set forth in the preamble of claim 1. Other thermographic devices are disclosed in US 5,776,074 and US 3,704,625.

There is therefore a need for an accurate analysis of these phenomena. An object of the present invention is to provide an instrument that allows an accurate and objective analysis of both described imperfections.

An object of the present invention is to provide an instrument which allows an accurate and objective analysis of both described imperfections and which is not complex to implement.

An object of the present invention is to provide an instrument which allows an accurate and objective analysis of both the described imperfections and which is simple to operate and use.

A further object of the present invention is to provide an instrument which allows an accurate and objective analysis of both described imperfections and at the same time allows monitoring the evolution of these imperfections over time.

### SUMMARY OF THE INVENTION

These and other objects are achieved by a contact thermography kit comprising a plurality of n temperature sensitive thermographic plates arranged to be placed in contact with a user, wherein each plate is adapted to assume a graphic pattern as a function of a detected temperature range so as to generate a corresponding thermographic image representative of the microcirculatory conditions of a user, wherein said plurality of plates is adapted to measure temperatures comprised between 24° and 36°, preferably between 26.5° and 34.6°.

The kit of the invention provides an instrument that allows an accurate and objective analysis of cellulite and adiposity.

In some embodiments, the kit comprises a temperature indicating unit arranged to indicate a choice of the plate to be used, and which provides a temperature indicator having n temperature sensitive fields comprised in said range between 24° and 36°.

The temperature indicating unit makes it easy to select the most suitable plate for the specific use.

In some embodiments, the kit comprises an image acquisition unit arranged to shoot said graphic pattern and acquire said thermographic image.

Preferably, the image acquisition unit is connected to a processing unit arranged for a graphic analysis of cellulite and adiposity on said thermographic image.

Preferably the processing unit is comprised in said image acquisition unit. In some embodiments, the kit comprises an electronic device comprising said image acquisition unit, wherein said electronic device is any mobile terminal, such as a tablet, a tablet PC, a personal computer or the like. The use of widely available electronic devices allows to keep the costs of the kit down and to make it easy to use.

In some embodiments, the kit comprises a structure adapted to simultaneously hold, in a predetermined reciprocal position, a plate on one side and the image acquisition unit on the other side.

The structure makes the use of the kit by the user considerably easier, guaranteeing the best possible quality of photographic detection of the thermographic image generated on the plate.

Preferably the kit comprises at least six thermochromic plates.

Six plates are an optimal solution to ensure the necessary detection accuracy over the desired temperature range, without overcomplicating the kit.

Preferably the plurality of thermochromic plates is adapted to measure temperatures comprised between 26.5° and 34.6° Celsius.

This range comprises all temperatures relevant to the analysis of cellulite and adiposity, while temperatures outside said range are not of interest for the purposes of the invention.

Preferably each thermochromic plate is configured to detect temperatures comprised within an range equal to about 3° C.

In some embodiments, the kit comprises six thermochromic plates L1-L6, configured to detect temperatures comprised in the respective ranges, as shown below:
L1 for temperatures in the range between 26.5°C and 29.6°C;
L2 for temperatures in the range between 27.5°C and 30.6°C;
L3 for temperatures in the range between 28.5°C and 31.6°C;
L4 for temperatures in the range between 29.5°C and 32.6°C;
L5 for temperatures in the range between 30.5°C and 33.6°C;
L6 for temperatures in the range between 31.5°C and 34.6°C.

In some embodiments, the kit comprises a request unit arranged to receive a request from the user for cellulite analysis or adiposity analysis. Preferably the temperature indicating unit, arranged to indicate a choice of the plate to be used, is connected to said request unit, and configured to indicate one or more plates usable for the requested analysis.

Preferably the processing unit comprises such a temperature indicating unit.

Preferably, the processing unit comprises an input module configured to receive said thermographic image acquired by said image acquisition unit. Preferably, the processing unit comprises a processing module configured to receive said thermographic image from said input module, compare it with an algorithm previously programmed during training step with previously classified thermographic images, and determine a corresponding classification.

Preferably, the processing unit is connected to a database of thermographic images comprising said previously classified thermographic images, wherein said images are classified into one of the following categories:
a. Cellulite absent
b. Oedematous cellulite
c. Fibrous cellulite
d. Sclerotic cellulite
e. Soft adiposity, in particular on the abdomen and/or arms and/or calves;
f. Hard or hardened adiposity, in particular on the abdomen and/or arms and/or calves.

Preferably, the processing module is configured to run an artificial intelligence algorithm previously generated by means of a training step starting from said previously classified thermographic images in order to assign said corresponding classification.

This solution allows an accurate and objective interpretation of the thermographic image, regardless of the user's experience and skills. Preferably the processing unit comprises an output module configured to output this classification.

Preferably, the processing unit comprises a feedback module configured to receive from a user a signal representative of an acceptance of said classification output by said output module or a signal representative of a request for a new analysis to replace or supplement the previous one.

The invention achieves the following technical effects:
- an accurate and objective indication of both described imperfections;
- non-complex implementation;
- simplicity of operation and use;
- comprehensive analysis of all body imperfections in particular on legs and/or arms and/or abdomen and/or calves;
- analysis of adiposity on legs and/or calves;
- objective, software-supported analysis;
- classification of cellulite stages and distinction of adiposity type to select targeted treatments;
- monitoring of the development of imperfections and chronological comparison before and after anti- imperfection treatments

The aforementioned and other technical effects/advantages of the invention will result in more detail from the description below of an example embodiment given by way of indication and not limitation with reference to the accompanying drawings.

### BRIEF DESCRIPTION OF THE DRAWINGS

Figure 1 is a schematic view of a contact thermography kit, according to the invention; and
Figure 2 is a schematic view of a contact thermography kit, according to the invention, in use.

### DETAILED DESCRIPTION

The kit of the invention provides an innovative means of assisted thermographic image analysis which allows carrying out thermographic analysis on legs, abdomen, arms and calves using high-resolution, reusable, temperature sensitive thermographic plates.

According to the invention, each user on an own electronic device will be able to have at his/her disposal the analysis application (or app) which allows not only to record his/her own data and the data of each thermographic analysis session, and to make chronological comparisons between different analyses, but above all to make an assisted interpretation of the analysis results.

The invention provides for a thermographic image to be acquired to start the analysis and to obtain, in a few seconds, indications of the level of cellulite or the type of adiposity present on the legs, abdomen, arms and calves.

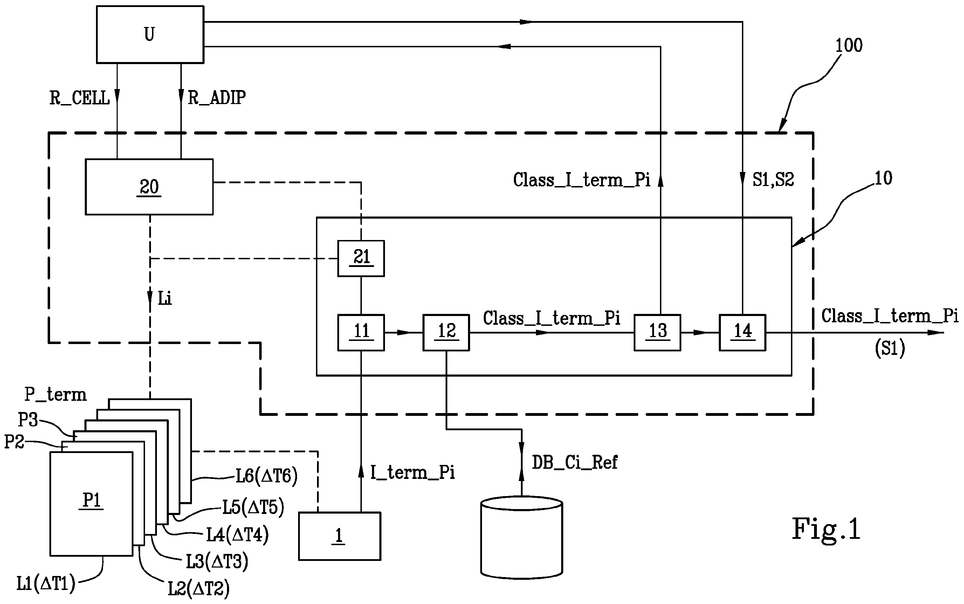

Referring to Figure 1, a contact thermography kit according to the invention comprises a plurality P_term of thermographic plates Li; (i=1..n); said plates are arranged to be placed in contact with parts of a user's body U, for example legs, abdomen, arms and calves.

In the course of the description, the term user will mean a person who can perform actions on himself or on a different subject.

In a preferred embodiment, the user is expected to be an operator performing actions on a different subject.

In a non-preferred alternative, the user is the subject who performs actions on himself.

According to the invention, each plate is adapted to assume a different graphical pattern Pi as a function of a detected temperature range ΔTi (i= 1..n), so as to generate a corresponding thermographic image I_term_Pi (i=1..n).

In other words, when the plate is placed on the body part, the plate detects temperatures of that part of the body and determines a corresponding graphic pattern.

According to the invention, the plurality P_term of thermochromic plates Li is variable in number and is adapted to measure temperatures comprised between 24° and 36° centigrade, preferably between 26.5° and 34.6° centigrade.

Preferably, each thermochromic plate is configured to detect temperatures comprised within a range equal to about 3°C (for example 3.1°C). Advantageously, the temperature ranges of the various plates overlap each other. In fact, the thermochromic response of each plate Li is best in the central part of this temperature range, since the colours that make up the graphic pattern are well distinct from each other. On the contrary, the quality of the thermochromic response tends to deteriorate towards the extremes of the range ΔTi, as the colours that make up the graphic pattern tend to saturate and blend together. For this reason, it may be useful to use another plate, for example the plate Li+1 or the plate Li-1. This observation of course does not apply to the first plate L1 and to the last plate Ln.

In particular, the plate L1 is configured to detect the lowest temperatures, comprised within the range ΔT1, among those considered of interest for the invention. For example, ΔT1 may extend from 26.5°C to 29.6°C. In the course of their experiments, the Applicant has found that temperatures below 26.5°C are not of interest for the purposes of the invention. Similarly, the plate Ln is configured to detect the highest temperatures, comprised within the range ΔTn, among those considered of interest to the invention. For example, ΔTn may extend from 31.5°C to 34.6°C. In the course of their experiments, the Applicant has found that temperatures above 34.6°C are not of interest for the purposes of the invention.

It is understood that the greater the number of thermochromic plates Li available, the better the quality of the overall thermochromic response over the entire temperature range.

Preferably the kit comprises at least six thermochromic plates Li (i=1 ...n). In a preferred embodiment, the kit comprises six thermochromic plates Li (i=1 ...6). Preferably, the six plates Li are configured to detect temperatures comprised in the respective ranges ΔTi, as shown below:
L1 for temperatures in the range ΔT1 between 26.5°C and 29.6°C;
L2 for temperatures in the range ΔT2 between 27.5°C and 30.6°C;
L3 for temperatures in the range ΔT3 between 28.5°C and 31.6°C;
L4 for temperatures in the range ΔT4 between 29.5°C and 32.6°C;
L5 for temperatures in the range ΔT5 between 30.5°C and 33.6°C;
L6 for temperatures in the range ΔT6 between 31.5°C and 34.6°C. Preferably the absolute values of the detectable temperatures will range from min (ΔT1)= 24° to max (ΔTn)= 36°, measured in degrees centigrade, even more preferably from min (ΔT1)= 26.5° to max (ΔTn)= 34.6°.

The plates Li used by the invention can be used on any part of the body.

In an embodiment of the invention, the kit comprises a temperature indicating unit 21 (also called ITS, or *Ideal Thermoplate Selector*), arranged to indicate a choice of the plate Li to be used, and which provides a temperature indicator having n temperature sensitive fields comprised in the described range between 24° and 36°, preferably between 26.5° and 34.6°.

Each field is calibrated to give a chromatic response as a function of the temperature of the area of the user's body U to be analysed.

This indicating unit performs a pre-analysis as it is used by placing it on the part of the user's body U to be analysed in order to identify the most suitable thermographic plate Li. The feedback is obtained following a colouring of one of the n fields of which it is constituted which will indicate precisely which plate Li, i = 1 ..n to use.

The temperature indicating unit 21 is therefore used indiscriminately for the selection of the suitable plate for carrying out an analysis of cellulite and adiposità.

With reference to figure 1, the system comprises a request unit 20 arranged to receive by the user U a request for cellulite analysis R_cell or adiposity analysis R_adip.

The request unit 20 comprises, in particular, a selection interface configured to allow a choice of the desired analysis.

In particular, the request unit is arranged in the electronic device 100 which will be described below.

In an embodiment, the request unit 20 is connected to the temperature indicating unit 21, arranged to indicate a choice of the plate to be used for the thermographic examination.

Preferably, the temperature indicating unit 21 is connected to an electronic processing unit 10 described below, to indicate one or more plates Li usable for the requested analysis R_cell, R_adip.

Indicatively, the plates that react to lower temperatures are best suited for thermographic measurements on the limbs, those that react to higher temperatures are best suited for measurements on the abdomen.

The kit comprises an image acquisition unit 1, in particular a photo module, arranged to shoot the graphic pattern Pi assumed by the plate Li and acquire the thermographic image I_term_Pi.

The image acquisition unit 1 is connected to an electronic processing unit 10 arranged for a graphic analysis of cellulite and adiposity on the thermographic image I_term_Pi.

The processing unit 10 is connected, in particular, remotely to the image acquisition unit 1.

Alternatively, the processing electronics unit 10 is comprised in the image acquisition unit 1.

In the course of the present description and subsequent claims, the electronic processing unit 10 is logically subdivided into distinct functional modules (memory modules or operating modules) that perform the described functions.

Such an electronic processing unit 10 may comprise a single electronic device, suitably programmed to perform the described functionalities, and the different modules may correspond to hardware entities and/or software routines forming part of the programmed device.

Alternatively or additionally, these functionalities may be performed by a plurality of electronic devices on which the aforesaid functional modules may be distributed.

The electronic processing unit 10 may also use one or more processors to execute the instructions contained in the memory modules; the aforesaid functional modules may also be distributed on different computers locally or remotely based on the architecture of the network in which they reside. In detail, the thermographic image I_term_Pi obtained by contact thermography is interpreted by the electronic processing unit 10 which detects the temperature variations typical of cellulite, classifying the main stages thereof (oedematous, fibrous, sclerotic) in order to select targeted treatments.

By way of example only, the following graphic patterns Pi can be detected for the following positionings of the plates:
- Uniform thermographic image and corresponding thermographic analysis of a user with no cellulite
- thermographic image with blurred spot pattern and corresponding thermographic analysis of oedematous cellulite, e.g., from position of plate on left upper thigh;
- leopard-skin thermographic image and corresponding to thermographic analysis of fibrous cellulite, e.g., from position of plate on right upper outer thigh;
- predominantly black-spotted thermographic image corresponding to thermographic analysis of sclerotic cellulite, e.g., from position of plate on frontal right thigh;
- leopard-skin and black-spotted thermographic image corresponding to thermographic analysis of fibrous and sclerotic cellulite, e.g., from position of plate on frontal left thigh.

The thermographic image I_term_Pi obtained by contact thermography is interpreted by the processing unit 10 in the adiposity analysis and makes it possible to detect and distinguish between soft and hard adipose tissue, and to accurately locate the areas of hard adipose tissue on which targeted and repeated actions are required.

Graphic patterns Pi can be detected relating to:
- thermographic image with different colours, some lighter and some darker, corresponding to thermographic analysis of discrete presence of mixed adipose tissue with hardening in the area indicated by the darker colour;
- thermographic image with brown coloured area corresponding to thermographic analysis of hard adipose tissue, e.g., from central arm area;
- thermographic analysis of slight presence of soft adipose tissue e.g., from position of plate on calf;
- thermographic analysis of the presence of soft adipose tissue e.g., from position of plate on the calf.

According to the invention, the kit comprising an electronic device 100 comprising the image acquisition unit 1.

The electronic device 100 according to the invention is any mobile terminal, in particular a tablet with iOS or Android system, a tablet PC, a personal computer or the like.

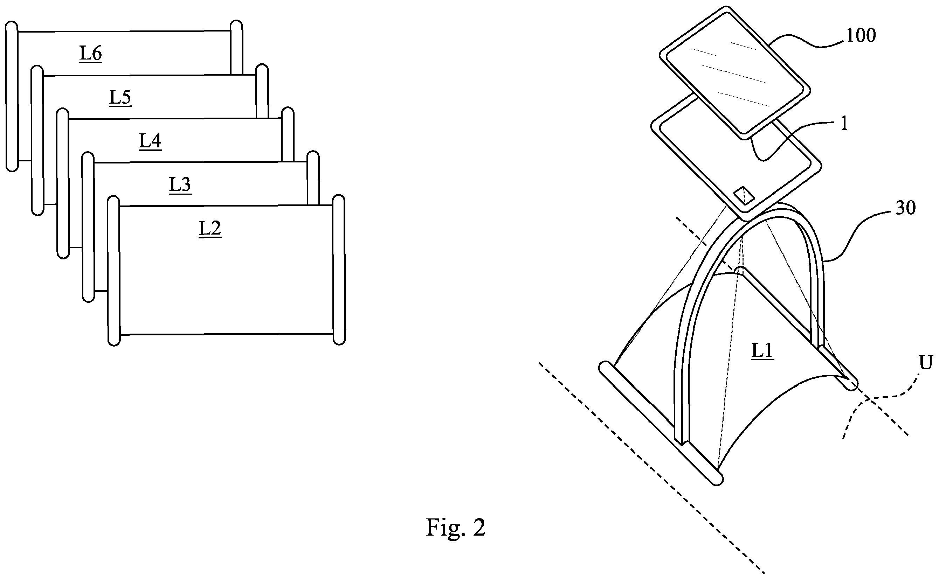

In accordance with some embodiments, the kit comprises a structure 30, shown schematically in figure 2. The structure 30 adapted to simultaneously hold, in a predetermined reciprocal position, a plate Li on one side, and the image acquisition unit 1, preferably the entire electronic device 100, on the other side.

Figure 2 shows in particular the kit during the use of the structure 30. The structure 30 holds the plate L1, which rests on the body of the user U, while the other plates L2-L6 remain available. For the sake of clarity of representation, the electronic device 100 comprising the image acquisition unit 1 is shown detached from its seat provided on the structure 30.

The structure 30, by ensuring that the plate Li and the image acquisition unit 1 assume a predefined reciprocal position in terms of distance and orientation, guarantees the best possible quality of photographic detection of the thermographic image generated on the plate. The use of structure 30 prevents problems deriving from a not optimal acquisition of the thermographic image, e.g., an acquisition too far away, too close, at the wrong angle and the like. In addition, the use of the structure 30 allows easier use of the kit by the user, whether he/she is operating on a different subject or on him/herself.

Once the user has carried out the photographic detection of the thermographic image generated on the plate, at the time of analysis, the user U can access the assisted interpretation function on the electronic device 100.

In other words, the invention provides for a digital acquisition of the thermographic plate at high resolution and for the acquired image to be then processed by an algorithm, in particular based on artificial intelligence, able to provide an interpretation referable to the stage of cellulite and type of found adiposity.

To this end, the processing unit is appropriately structured as described below, with reference to the figure.

The processing unit 10 comprises an input module 11 configured to receive the thermographic image I_term_Pi acquired by the image acquisition unit 1.

At this point, an app running on the electronic device queries an algorithm that receives the thermographic image as input and produces a classification of cellulite and adiposity as output.

In other words, with reference to the figure, the processing unit 10 comprises a processing module 12 configured to receive the thermographic image I_term_Pi from the input module 11, use it as a query input of a mathematical model previously processed on the basis of a database of previously classified thermographic images DB_I_term I_term_Pi_pred and determine a corresponding classification Class_I_term_Pi.

The processing unit 10 is connected to a database of thermographic images DB_I_term comprising the aforesaid previously classified thermographic images I_term_Pi_pred.

According to the invention, the images are classified into one of the following categories:
a. Cellulite absent;
b. Oedematous cellulite;
c. Fibrous cellulite;
d. Sclerotic cellulite;
e. Soft adiposity in particular on the abdomen and/or arms and/or calves;
f. Hard adiposity in particular on the abdomen and/or arms and/or calves.

According to the invention, the processing module 12 is configured to run an artificial intelligence algorithm Alg and to assign the corresponding classification Class_I_term_Pi.

The algorithm Alg was previously developed through a training step starting from the previously classified thermographic images I_term_Pi_pred and is subsequently queried to assign the aforesaid classification to each new analysis. The achievement of a new analysis with relative classification becomes a new training element for the algorithm Alg so that it can be refined with each query.

The processing unit 10 comprises an output module 13 configured to output the assigned classification Class_I_term_Pi and send it to the user U.

The electronic device 100 returns as output to the user U who made the request, the determined classification and offers the user the possibility to accept or modify the feedback obtained. In particular, the user U has a drop-down menu at his/her disposal which allows to confirm or reject the assisted interpretation provided by the output module 13.

The processing unit 10 comprises a feedback module 14 configured to receive from a user U a signal S1 representative of an acceptance of the classification Class_I_term_Pi output by said output module 13 or a signal S2 representative of a request for a new analysis which may replace the previous one or supplement it thus allowing the realisation of a collection of analyses carried out on several parts of the body of the user U.

In addition, the feedback module 14 is configured to retrieve previously archived analyses, thus allowing a chronological comparison thereof and at the same time an objective support to the improvement of the microcirculatory conditions of the user U after targeted treatments.

An inventive kit based on contact thermography for the analysis of cellulite and adiposity has been described.

The invention achieves the following technical effects:
- an accurate and objective indication of both the described imperfections
- non-complex implementation;
- simplicity of operation and use;
- comprehensive analysis of all body imperfections in particular on legs and/or arms and/or abdomen and/or calves;
- analysis of adiposity on arms and/or calves;
- objective, software-supported analysis;
- classification of cellulite stages and distinction of adiposity type to select targeted treatments;
- historical comparison of analyses to objectively support the effects deriving from any targeted treatments undertaken as a result of thermographic analyses.

## Claims

1. A contact thermography kit comprising a plurality (P_term) of thermochromic plates (Li; i=1..n) arranged to be placed in contact with a user (U), wherein each plate (Li) is adapted to assume a graphic pattern (Pi) as a function of a detected temperature range (ΔTi; i = 1..n), so as to generate a corresponding thermographic image (I_term_Pi; i=1..n), wherein said plurality (P_term) of plates (Li) is adapted to measure temperatures comprised between 26.5° and 34.6° centigrade, **characterized in that** the plate (L1) configured to detect the lowest temperatures is configured to detect temperatures comprised within the range from 26.5°C to 29.6°C.

2. The kit according to claim 1 comprising a temperature indicating unit (21), arranged to indicate a choice of the plate (Li) to be used, and which provides a temperature indicator having n temperature sensitive fields comprised in said range between 26.5° and 34.6°.

3. The kit according to claim 1 or 2 comprising an image acquisition unit (1) arranged to shoot said graphic pattern (Pi) and acquire said thermographic image (I_term_Pi; i=1..n).

4. The kit according to claim 3 wherein said image acquisition unit (1) is connected to a processing unit (10) arranged for a graphic analysis of cellulite and adiposity on said thermographic image (I_term_Pi).

5. The kit according to any one of claims 3 to 4 comprising an electronic device (100) comprising said image acquisition unit (1), wherein said electronic device (100) is any mobile terminal, such as a tablet, a tablet PC, a personal computer or the like.

6. The kit according to any one of claims 3 to 5, further comprising a structure (30) adapted to simultaneously hold, in a predetermined reciprocal position, a plate (Li) on one side and the image acquisition unit (1) on the other side.

7. The kit according to any of the preceding claims, comprising at least six thermochromic plates (Li; i=1..n).

8. The kit according to any one of the preceding claims, wherein each thermochromic plate (Li) is configured to detect temperatures comprised within a range (ΔTi) equal to about 3° C.

9. The kit according to any one of the preceding claims, comprising six thermochromic plates (Li; i=1..6) and wherein the six plates (Li) are configured to detect temperatures comprised in the respective ranges (ΔTi), as reported below:
L1 for temperatures in the range ΔT1 between 26.5°C and 29.6°C;
L2 for temperatures in the range ΔT2 between 27.5°C and 30.6°C;
L3 for temperatures in the range ΔT3 between 28.5°C and 31.6°C;
L4 for temperatures in the range ΔT4 between 29.5°C and 32.6°C;
L5 for temperatures in the range ΔT5 between 30.5°C and 33.6°C;
L6 for temperatures in the range ΔT6 between 31.5°C and 34.6°C.

10. The kit according to any one of claims 4 to 9 wherein said one processing unit (10) comprises an input module (11) configured to receive said thermographic image (I_term_Pi) acquired by said image acquisition unit (1), and a processing module (12) configured to receive said thermographic image (I_term_Pi) from said input module (11), compare it with an algorithm previously programmed during training step with previously classified thermographic images (I_term_Pi_pred), and determine a corresponding classification (Class_I_term_Pi).

11. The kit according to claim 10 wherein said processing unit (10) is connected to a database of thermographic images (DB_I_term) comprising said previously classified thermographic images (I_term_Pi_pred), wherein said images are classified into one of the following categories:
a. Cellulite absent
b. Oedematous cellulite
c. Fibrous cellulite
d. Sclerotic cellulite
e. Soft adiposity, in particular on the abdomen and/or arms and/or calves;
f. Hard adiposity, in particular on the abdomen and/or arms and/or calves.

12. The kit according to any one of claims 10 to 11 wherein said processing module (12) is configured to run an artificial intelligence algorithm (Alg) previously generated by means of a training step starting from said previously classified thermographic images (I_term_Pi_pred) in order to assign said corresponding classification (Class_I_term_Pi).

13. The kit according to any one of claims 10 to 12 wherein said processing unit (10) comprises an output module (13) configured to output said classification (Class_I_term_Pi), and wherein said processing unit (10) comprises a feedback module (14) configured to receive from a user (U) a signal (S1) representative of an acceptance of said classification (Class_I_term_Pi) output by said output module (13) or a signal (S2) representative of a request for a new analysis to replace or supplement the previous one.

## Patentansprüche

1. Ein Kontaktthermografie-Kit, umfassend eine Vielzahl (P_term) von thermochromen Platten (Li; i=1..n), die so angeordnet sind, dass sie in Kontakt mit einem Benutzer (U) gebracht werden, wobei jede Platte (Li) dazu eingerichtet ist, ein grafisches Muster (Pi) als Funktion eines erfassten Temperaturbereichs (ΔTi; i=1..n) ein grafisches Muster (Pi) anzunehmen, um ein entsprechendes thermografisches Bild (I_term_Pi; i=1..n) zu erzeugen, wobei die Vielzahl (P _term) von Platten (Li) dazu eingerichtet ist, Temperaturen zwischen 26.5° und 34.6° Celsius zu messen,
**dadurch gekennzeichnet, dass** die Platte (L1), die zum Erfassen der niedrigsten Temperaturen konfiguriert ist, dazu konfiguriert ist, Temperaturen innerhalb des Bereichs von 26,5 °C bis 29,6 °C zu erfassen.

2. Das Kit nach Anspruch 1, umfassend eine Temperaturanzeigeeinheit (21), die dazu eingerichtet ist, eine Wahl der zu verwendenden Platte (Li) anzuzeigen, und die einen Temperaturindikator mit n temperaturempfindlichen Feldern bereitstellt, die in dem genannten Bereich zwischen 26.5° und 34.6° enthalten sind.

3. Das Kit nach Anspruch 1 oder 2, umfassend eine Bilderfassungseinheit (1), die dazu eingerichtet ist, das grafische Muster (Pi) aufzunehmen und das thermografische Bild (I_term_Pi; i=1..n) zu erfassen.

4. Das Kit nach Anspruch 3, wobei die Bilderfassungseinheit (1) mit einer Verarbeitungseinheit (10) verbunden ist, die für eine grafische Analyse von Cellulite und Adipositas auf dem thermografischen Bild (I_term_Pi) eingerichtet ist.

5. Das Kit nach einem der Ansprüche 3 bis 4, umfassend ein elektronisches Gerät (100), welches die Bilderfassungseinheit (1) umfasst, wobei das elektronische Gerät (100) ein beliebiges mobiles Endgerät, wie ein Tablet, ein Tablet-PC, ein Personal Computer oder dergleichen ist.

6. Das Kit nach einem der Ansprüche 3 bis 5, ferner umfassend eine Struktur (30), die dazu eingerichtet ist, eine Platte (Li) auf einer Seite und die Bilderfassungseinheit (1) auf der anderen Seite gleichzeitig in einer vorbestimmten wechselseitigen Position zu halten.

7. Das Kit nach einem der vorhergehenden Ansprüche, umfassend mindestens sechs thermochrome Platten (Li; i=1..n).

8. Das Kit nach einem der vorhergehenden Ansprüche, wobei jede thermochrome Platte (Li) dazu eingerichtet ist, Temperaturen innerhalb eines Bereichs (ΔTi) entsprechend etwa 3 °C zu erfassen.

9. Das Kit nach einem der vorhergehenden Ansprüche, umfassend sechs thermochrome Platten (Li; i=1..6) und wobei die sechs Platten (Li) dazu konfiguriert sind, dass sie Temperaturen, die in den jeweiligen Bereichen (ΔTi) wie unten angegeben enthalten sind, erfassen:
L1 für Temperaturen im Bereich ΔT1 zwischen 26.5°C und 29.6°C;
L2 für Temperaturen im Bereich ΔT2 zwischen 27.5°C und 30.6°C;
L3 für Temperaturen im Bereich ΔT3 zwischen 28.5°C und 31.6°C;
L4 für Temperaturen im Bereich ΔT4 zwischen 29.5°C und 32.6°C;
L5 für Temperaturen im Bereich ΔT5 zwischen 30.5°C und 33.6°C;
L6 für Temperaturen im Bereich ΔT6 zwischen 31.5°C und 34.6°C.

10. Das Kit nach einem der Ansprüche 4 bis 9, wobei besagte eine Verarbeitungseinheit (10) ein Eingabemodul (11), das dazu konfiguriert ist, das von der Bilderfassungseinheit (1) erfasste thermografische Bild (I_term_Pi) zu empfangen, und ein Verarbeitungsmodul (12) umfasst, das dazu konfiguriert ist, das thermografische Bild (I_term_Pi) von dem Eingabemodul (11) zu empfangen, es mit einem Algorithmus zu vergleichen, der zuvor während eines Trainingsschritts mit zuvor klassifizierten thermografischen Bildern (I_term_Pi_pred) programmiert wurde, und eine entsprechende Klassifizierung (Class_I_term_Pi) zu bestimmen.

11. Das Kit nach Anspruch 10, wobei die Verarbeitungseinheit (10) mit einer Datenbank thermografischer Bilder (DB_I_term) verbunden ist, welche die zuvor klassifizierten thermografischen Bilder (I_term_Pi_pred) umfasst, wobei besagte Bilder in eine der folgenden Kategorien klassifiziert sind:
a. Cellulite nicht vorhanden
b. Ödematöse Cellulitis
c. Faserige Cellulitis
d. Sklerotische Cellulitis
e. Weiche Adipositas, insbesondere am Bauch und/oder an den Armen und/oder Waden;
f. Starke Adipositas, insbesondere am Bauch und/oder an den Armen und/oder Waden.

12. Das Kit nach einem der Ansprüche 10 bis 11, wobei das Verarbeitungsmodul (12) dazu konfiguriert ist, einen Algorithmus der künstlichen Intelligenz (Alg) auszuführen, der zuvor durch einen Trainingsschritt ausgehend von den zuvor klassifizierten thermografischen Bildern (I_term_Pi_pred) erzeugt wurde, um die entsprechende Klassifizierung (Class_I_term_Pi) zuzuweisen.

13. Das Kit nach einem der Ansprüche 10 bis 12, wobei die Verarbeitungseinheit (10) ein Ausgabemodul (13) umfasst, das dazu konfiguriert ist, die Klassifizierung (Class_I_term_Pi) auszugeben, und wobei die Verarbeitungseinheit (10) ein Rückmeldungsmodul (14) umfasst, das dazu konfiguriert ist, von einem Benutzer (U) ein Signal (S1) zu empfangen, das repräsentativ für ein Akzeptieren der von dem Ausgabemodul (13) ausgegebenen Klassifizierung (Class_I_term_Pi) ist, oder ein Signal (S2), das für eine Anforderung einer neuen Analyse repräsentativ ist, welche die vorherige ersetzen oder ergänzen soll.

## Revendications

1. - Kit de thermographie de contact comprenant une pluralité (P_term) de plaques thermochromiques (Li ; i=1...n) agencées pour être placées en contact avec un utilisateur (U), chaque plaque (Li) étant apte à adopter un motif graphique (Pi) en fonction d'une plage de température détectée (Δti ; i=1...n), de façon à générer une image thermographique correspondante (I_term_Pi ; i=1...n), ladite pluralité (P_term) de plaques (Li) étant aptes à mesurer des températures comprises entre 26,5° et 34,6° centigrades, **caractérisé par le fait que** la plaque (L1) configurée pour détecter les températures les plus basses est configurée pour détecter des températures comprises dans la plage de 26,5°C à 29,6°C.

2. - Kit selon la revendication 1, comprenant une unité d'indication de température (21), agencée pour indiquer un choix de la plaque (Li) à utiliser, et qui fournit un indicateur de température ayant n champs sensibles à la température, compris dans ladite plage entre 26,5° et 34,6°.

3. - Kit selon la revendication 1 ou 2, comprenant une unité d'acquisition d'image (1) agencée pour photographier ledit motif graphique (Pi) et acquérir ladite image thermographique (I_term_Pi ; i=1...n).

4. - Kit selon la revendication 3, dans lequel ladite unité d'acquisition d'image (1) est connectée à une unité de traitement (10) agencée pour une analyse graphique de cellulite et d'adiposité sur ladite image thermographique (I_term_Pi).

5. - Kit selon l'une quelconque des revendications 3 à 4, comprenant un dispositif électronique (100) comprenant ladite unité d'acquisition d'image (1), ledit dispositif électronique (100) étant un terminal mobile quelconque, tel qu'une tablette, un ordinateur tablette, un ordinateur personnel ou analogue.

6. - Kit selon l'une quelconque des revendications 3 à 5, comprenant en outre une structure (30) agencée pour maintenir simultanément, dans une position réciproque prédéterminée, une plaque (Li) sur un côté et l'unité d'acquisition d'image (1) sur l'autre côté.

7. - Kit selon l'une quelconque des revendications précédentes, comprenant au moins six plaques thermochromiques (Li ; i=1...n).

8. - Kit selon l'une quelconque des revendications précédentes, dans lequel chaque plaque thermochromique (Li) est configurée pour détecter des températures comprises dans une plage (ΔTi) égale à environ 3°C

9. - Kit selon l'une quelconque des revendications précédentes, comprenant six plaques thermochromiques (Li ; i=1...6) et les six plaques (Li) étant configurées pour détecter des températures comprises dans les plages respectives (ΔTi), comme indiqué ci-dessous :
L1 pour des températures comprises dans la plage ΔT1 entre 26,5°C et 29,6°C ;
L2 pour des températures comprises dans la plage ΔT2 entre 27,5°C et 30,6°C ;
L3 pour des températures comprises dans la plage ΔT3 entre 28,5°C et 31,6°C ;
L4 pour des températures comprises dans la plage ΔT4 entre 29,5°C et 32,6°C ;
L5 pour des températures comprises dans la plage ΔT5 entre 30,5°C et 33,6°C ;
L6 pour des températures comprises dans la plage ΔT6 entre 31,5°C et 34,6°C.

10. - Kit selon l'une quelconque des revendications 4 à 9, dans lequel ladite unité de traitement (10) comprend un module d'entrée (11) configuré pour recevoir ladite image thermographique (I_term_Pi) acquise par ladite unité d'acquisition d'image (1), et un module de traitement (12) configuré pour recevoir ladite image thermographique (I_term_Pi) en provenance dudit module d'entrée (11), la comparer, à l'aide d'un algorithme préalablement programmé lors d'une étape d'apprentissage, à des images thermographiques préalablement classées (I_term_Pi_pred), et déterminer une classification correspondante (Class_I_term_Pi).

11. - Kit selon la revendication 10, dans lequel ladite unité de traitement (10) est connectée à une base de données d'images thermographiques (DB_I_term) comprenant lesdites images thermographiques préalablement classées (I_term_Pi_pred), lesdites images étant classées dans l'une des catégories suivantes :
a. Cellulite absente
b. Cellulite oedémateuse
c. Cellulite fibreuse
d. Cellulite scléreuse
e. Adiposité molle, en particulier sur l'abdomen et/ou les bras et/ou les mollets ;
f. Adiposité dure, en particulier sur l'abdomen et/ou les bras et/ou les mollets.

12. - Kit selon l'une quelconque des revendications 10 à 11, dans lequel ledit module de traitement (12) est configuré pour exécuter un algorithme d'intelligence artificielle (Alg) préalablement généré au moyen d'une étape d'apprentissage commençant à partir desdites images thermographiques préalablement classées (I_term_Pi_pred) afin d'attribuer ladite classification correspondante (Class_I_term_Pi).

13. - Kit selon l'une quelconque des revendications 10 à 12, dans lequel ladite unité de traitement (10) comprend un module de sortie (13) configuré pour délivrer en sortie ladite classification (Class_I_term_Pi), et ladite unité de traitement (10) comprenant un module de retour (14) configuré pour recevoir, à partir d'un utilisateur (U), un signal (S1) représentant une acceptation de ladite classification (Class_I_term_Pi) délivrée en sortie par ledit module de sortie (13) ou un signal (S2) représentant une demande d'une nouvelle analyse en remplacement ou en complément de la précédente.
